# EUROPEAN PATENT APPLICATION

(11) **EP 4 357 770 A1**
(43) Date of publication of application: **24.04.2024**
(21) Application number: 23192055.4
(22) Date of filing: 18.08.2023
(51) Int. Cl.: G01N 27/414, B01L 3/00, G01N 33/00, G01N 27/64

(54) **ION-SENSITIVE FIELD EFFECT TRANSISTOR ABOVE MICROFLUIDIC CAVITY FOR ION DETECTION AND IDENTIFICATION**

(30) Priority: 18.10.2022 US 202218047405
(71) Applicant: GlobalFoundries U.S. Inc., Malta, NY 12020 (US)
(72) Inventor: Pawlak, Bartlomiej J., 3001 Leuven (BE); Levy, Mark D., Essex Junction, 05452 (US); Adusumilli, Siva P., Essex Junction, 05452 (US); Hazbun, Ramsey M., Essex Junction, 05452 (US)
(74) Representative: Grünecker Patent- und Rechtsanwälte PartG mbB

(57) **Abstract**

A structure (100) includes a cavity (140) in a semiconductor substrate (108); a field effect transistor (112) positioned over the cavity (140); an opening (130) in the semiconductor substrate (108) extending to the cavity (140); and a layer of insulating material (132) filling the opening (130) and forming an insulating material window (132) to the cavity (140).

## Description

### BACKGROUND

### Field of the Invention

The present invention relates to semiconductor structures and, more particularly, to structure embodiments including a sensor and to method embodiments for forming the structure and for operating the sensor.

### Related Art

Sensors based on ion-sensitive field effect transistors (ISFETs) can be integrated into chip manufacturing processes and can be used to detect and measure various aspects of chemical reactions and substance properties. For example, an ISFET may be used as a sensor in an integrated circuit to measure ion concentrations, such as hydrogen ion concentration, in a sample of an analyte solution.

### SUMMARY

Aspects of the disclosure include a structure including: a cavity in a semiconductor substrate; an ion-sensitive field effect transistor (ISFET) positioned over the cavity; an opening in the semiconductor substrate extending to the cavity; and a layer of insulating material filling the opening and forming an insulating material window to the cavity.

Aspects of the disclosure further include a method, including: directing light at a predetermined frequency through an insulating material window into a cavity in a semiconductor substrate, wherein the cavity contains a sample; and sensing a source to drain current of an ion-sensitive field effect transistor (ISFET) positioned over the cavity while directing the light at the predetermined frequency through the insulating material window into the cavity.

Aspects of the disclosure further include a structure comprising: a plurality of cavities in a semiconductor substrate; a plurality of ion-sensitive field effect transistors (ISFETs) wherein each ISFET of the plurality of ISFETS is positioned over a respective cavity of the plurality of cavities; and at least one insulating material window extending to the plurality of cavities, wherein the at least one insulating material window directs light from at least one light source into the plurality of cavities.

### BRIEF DESCRIPTION OF THE DRAWINGS

The present invention will be better understood from the following detailed description with reference to the drawings, which are not necessarily drawn to scale. In the drawings, like reference numerals refer to like features in the various views.
FIG. 1 depicts a semiconductor structure for a sensor including an ion-sensitive field effect transistor (ISFET) above a microfluidic cavity with an insulating material window formed over the microfluidic cavity in accordance with embodiments of the disclosure.
FIG. 2 depicts a semiconductor structure for a sensor including an ISFET above a microfluidic cavity with an insulating material window formed below the microfluidic cavity in accordance with embodiments of the disclosure.
FIG. 3 depicts a semiconductor structure for a sensor including an ISFET above a microfluidic cavity with an insulating material window formed to the side of the microfluidic cavity in accordance with embodiments of the disclosure.
FIGS. 4 and 5 depicts a process for ion detection an identification carried out by the semiconductor structure of FIG. 1 according to embodiments of the disclosure.
FIGS. 6-11 depict an illustrative process for forming the semiconductor structure of FIG. 1 according to embodiments of the disclosure.
FIGS. 12-18 depict an illustrative process for forming the semiconductor structure of FIG. 3 according to embodiments of the disclosure.
FIG. 19 depicts a semiconductor structure including a plurality of ISFETS and a microfluidic network according to embodiments of the disclosure.
FIG. 20 depicts a semiconductor structure including a plurality of ISFETS and a microfluidic network according to embodiments of the disclosure.

### DETAILED DESCRIPTION

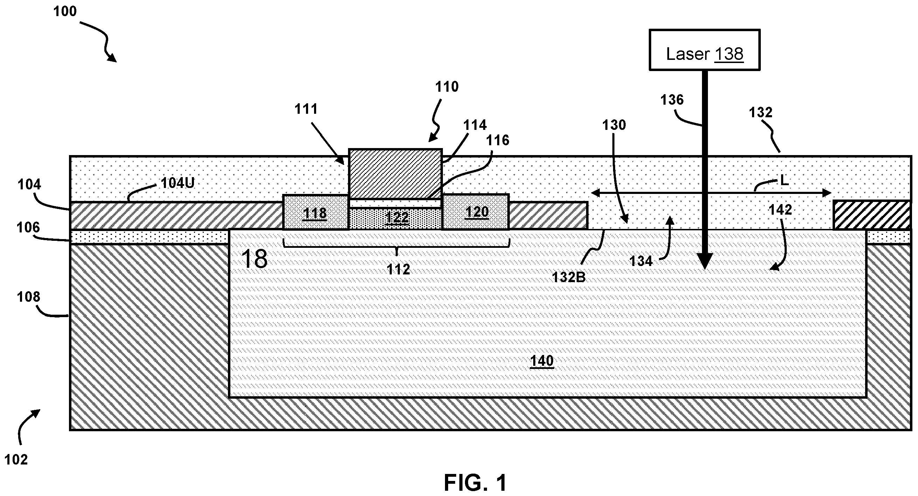

With reference to FIG. 1 and in accordance with embodiments of the disclosure, a structure 100 for a sensor is formed on a wafer 102. In an embodiment, the wafer 102 may be a semiconductor-on-insulator (SOI) wafer. The wafer 102 may include a device layer 104 used to fabricate an ISFET 110 or other suitable sensor sensitive to ions such as a bioFET, an insulating layer 106 (e.g., in the form of a buried oxide (BOX) layer), and a substrate 108. As shown, the device layer 104 is separated from the substrate 108 by the intervening insulating layer 106 and is considerably thinner than the substrate 108. The device layer 104 and the substrate 108 may be composed of a single-crystal semiconductor material, such as monocrystalline silicon or other suitable substrate material. The insulating layer 106 may be formed of silicon dioxide or other suitable insulating material. In an embodiment, the wafer 102 may be an extremely thin semiconductor-on-insulator (ETSOI) wafer with a thin device layer 104 and a thin buried insulating layer 106, and may be used to fabricate the ISFET 110 as a fully-depleted SOI (FDSOI) device.

The ISFET 110 may be fabricated by front-end-of-line (FEOL) processing in an active region 112 of the device layer 104. According to some embodiments, as depicted in FIG. 1, the ISFET 110 may include a planar gate 111 including a gate electrode 114 and a gate dielectric 116. The gate electrode 114 may be composed of a conductor, such as doped polycrystalline silicon (i.e., polysilicon) or other suitable material, and the gate dielectric 116 may be composed of an electrical insulator, such as silicon dioxide, silicon nitride, or other suitable insulating material.

The ISFET 110 may further include, within the active region 112 of the device layer 104, a source region 118, a drain region 120, and a channel region 122 positioned laterally between the source and drain regions 118, 120. According to some embodiments of the disclosure, the ISFET 110 may not include the planar gate 111. To this extent, the ISFET 110 includes the source region 118, the drain region 120, and the channel region 122 positioned laterally between the source and drain regions 118, 120.

In the below description, reference is made to regions of the ISFET 110 doped so as to have a first-type conductivity or a second-type conductivity that is different from the first-type conductivity. It should be understood that the first-type conductivity and the second-type conductivity are either P-type conductivity and N-type conductivity, respectively, or N-type conductivity and P-type conductivity, respectively, depending upon whether the ISFET is an N-type ISFET (N-ISFET) or a P-type ISFET (P-ISFET). According to embodiments of the disclosure, the ISFET 110 may be either an N-ISFET or a P-ISFET. For example, if the ISFET 110 is an N-ISFET, then the first-type conductivity refers to P-type conductivity and the second-type conductivity refers to N-type conductivity. However, if the ISFET 110 is a P-ISFET, then the first-type conductivity refers to N-type conductivity and the second-type conductivity refers to P-type conductivity. Thus, in the ISFET 110, the channel region 122 can have the first-type conductivity at a relative low conductivity (or alternatively can be undoped) and the source region 118 and the drain region 120 can have the second-type conductivity at a relatively high conductivity level. For example, for an N-ISFET, the channel region 122 can be a P- channel region 122 and the source region 118 and the drain region 120 can be N+ source/drain regions. For a P-ISFET, the channel region 122 can be an N- channel region and the source region 118 and the drain region 120 can be P+ source/drain regions. See the detailed discussion below regarding different dopants that can be employed in semiconductor materials to achieve P-type conductivity or N-type conductivity.

According to embodiments of the disclosure, a portion of the device layer 104 of the wafer 102 may be removed (e.g., during back-end-of-line (BEOL) processing) to form an opening 130 in the device layer 104. A layer of insulating material 132 may then be formed over the ISFET 110, the upper surfaces 104U of the device layer 104, and the opening 130 in the device layer 104. The insulating material 132 may be formed of silicon dioxide, silicon nitride, or other suitable insulating material, using a conformal deposition process or the like. As shown in FIG. 1, the insulating material 132 completely fills the opening 130 in the device layer 104.

A microfluidic cavity 140 may be formed in selected portions of the wafer 102. For example, the microfluidic cavity 140 may be formed by selectively removing portions of the substrate 108 and the insulating layer 106 below the ISFET 110 and the opening 130 in the device layer 104. A back-side etch of the wafer 102 (e.g., during BEOL processing) may be performed to form the microfluidic cavity 140.

As shown in FIG. 1, the microfluidic cavity 140 extends through the substrate 108 and the insulating layer 106 of the wafer 102 to a bottom surface of the source, drain, and channel regions 118, 120, 122 of the ISFET 110. Further, the microfluidic cavity 140 extends laterally below and beyond a bottom surface 132B of the insulating material 132 filling the opening 130 in the device layer 104. As will be described in greater detail below, according to embodiments of the disclosure, the insulating material 132 within the opening 130 forms an insulating material window 134 that allows light 136 from a light source 138 (e.g., a tunable external laser) to enter the microfluidic cavity 140 to allow excitation spectroscopy to be performed on ions in a sample 142 (e.g., fluid or gas) contained and/or flowing through the microfluidic cavity 140. The light source 138 may be tunable and may provide light 136 at different frequencies and excitation energies. For example, the light source 138 may be configured to provide light at various frequencies across a range of the visible light spectrum. According to embodiments of the disclosure, the length L of the insulating material window 134 should be larger than the wavelength of the light 136. For example, the length L of the insulating material window 134 may be from about 2000 nms to about 1-2 mms in length.

Various lithography and etching processes may be employed to form the opening 130 and the microfluidic cavity 140. For example, the lithography process may entail forming an etch mask that includes a layer of a light-sensitive material, such as an organic photoresist, applied by a spin coating process, pre-baked, exposed to light projected through a photomask, baked after exposure, and developed with a chemical developer to form an opening over the intended location for the opening 130 and microfluidic cavity 140. The etching process may be a reactive ion etching process, and the etch mask may be stripped by, for example, plasma ashing, followed by a cleaning process.

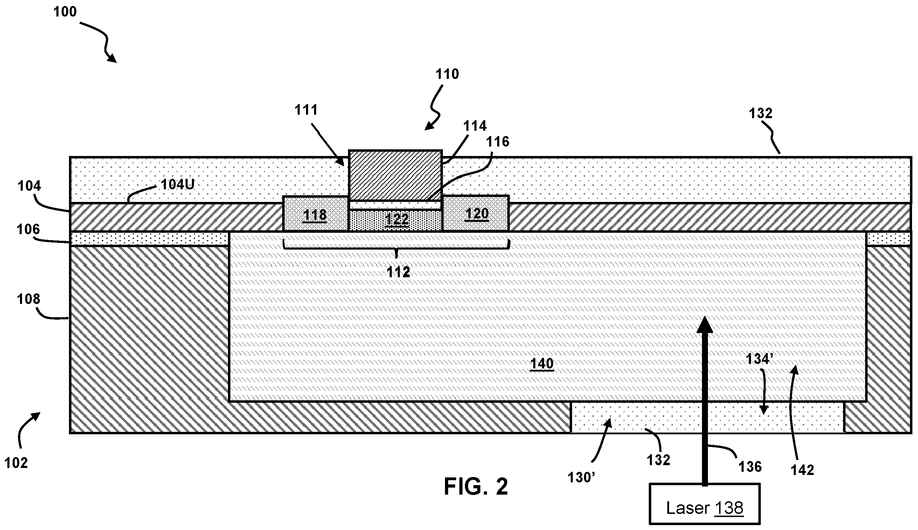

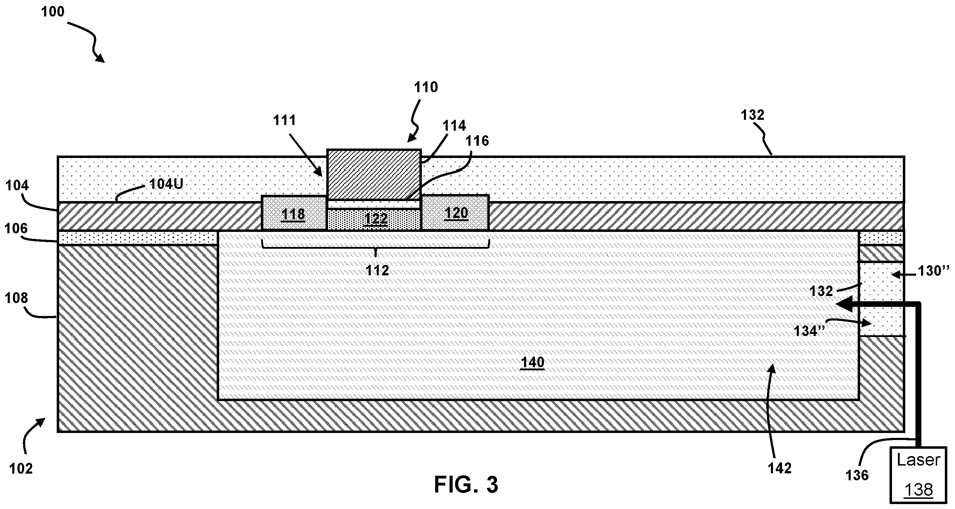

In the structure depicted in FIG. 1, the insulating material window 134 is positioned to allow light 136 from the light source 138 to enter the microfluidic cavity 140 from a top side of the structure 100. According to additional embodiments of the disclosure, the insulating material window 134 may also be formed such that light 136 from the light source 138 may enter the microfluidic cavity 140 from a bottom side of the structure 100 (FIG. 2) or from a side of the structure 100 (FIG. 3). In FIG. 2, for example, the substrate 108 may be suitably etched to form an opening 130' on the bottom of the structure 100 that is subsequently filled with an insulating material 132 to form an insulating material window 134' that allows light 136 from the light source 138 to enter the microfluidic cavity 140 from the bottom of the structure 100. Similarly, in FIG. 3, the substrate 108 may be suitably etched to form an opening 130" on the side of the structure 100. The opening 130" is subsequently filled with an insulating material 132 to form an insulating material window 134" that allows light 136 from the light source 138 to enter the microfluidic cavity 140. Other locations for the insulating material window 134 are also possible.

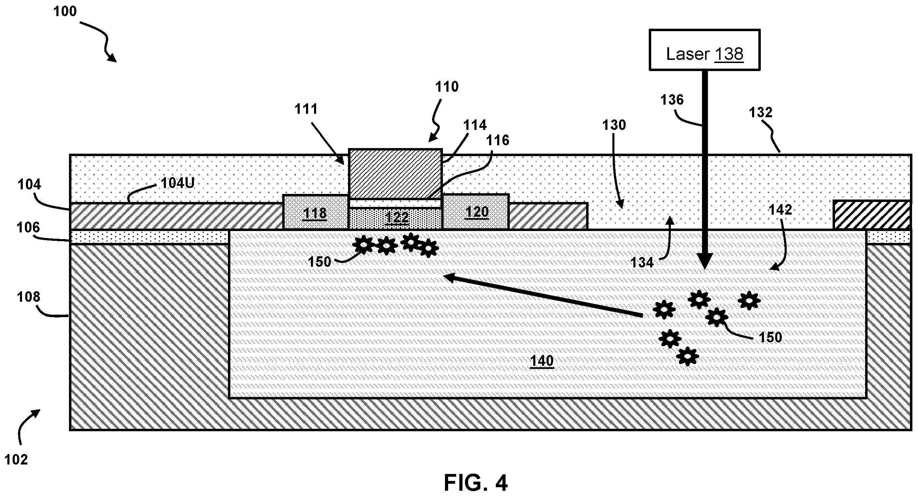

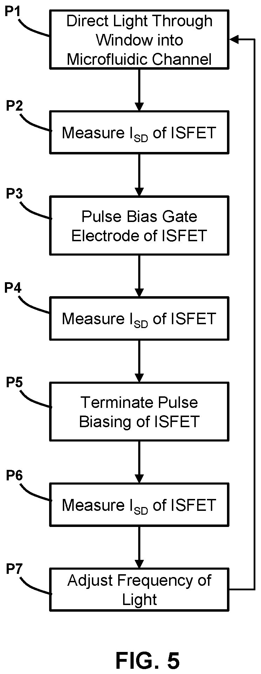

A method for ion detection and identification according to embodiments is presented with reference to FIGS. 4 and 5. Initially, this method may be performed using a sample 142 containing a known molecule of interest to determine the correlation between the excitation energy of the light 136 produced by the light source 138 and the source to drain current I_{SD} at the ISFET 110 due to ionization of the molecule. This method may be performed for a plurality of different molecules. Thereafter, identification of a molecule in the sample 142 may be obtained based on the measured source to drain current I_{SD} and the excitation energy(ies) of the light 136 output by the light source 138.

At process P1, the light source 138 directs light 136 at a predetermined frequency and excitation energy through the insulation material window 134 into the microfluidic cavity 140, exciting and ionizing molecules within the sample 142 and producing ions 150. For example, light 136 at a first frequency may excite a first type of ion 150, while light 136 at a second, different frequency may excite a second, different type of ion 150. The ions 150 in the sample 142 act as a gate electrode for the ISFET 110 and, depending on the concentration of ions 150 in the sample 142, cause a current to flow from the source region 120 to the drain region 118 (I_{SD}) of the ISFET 110.

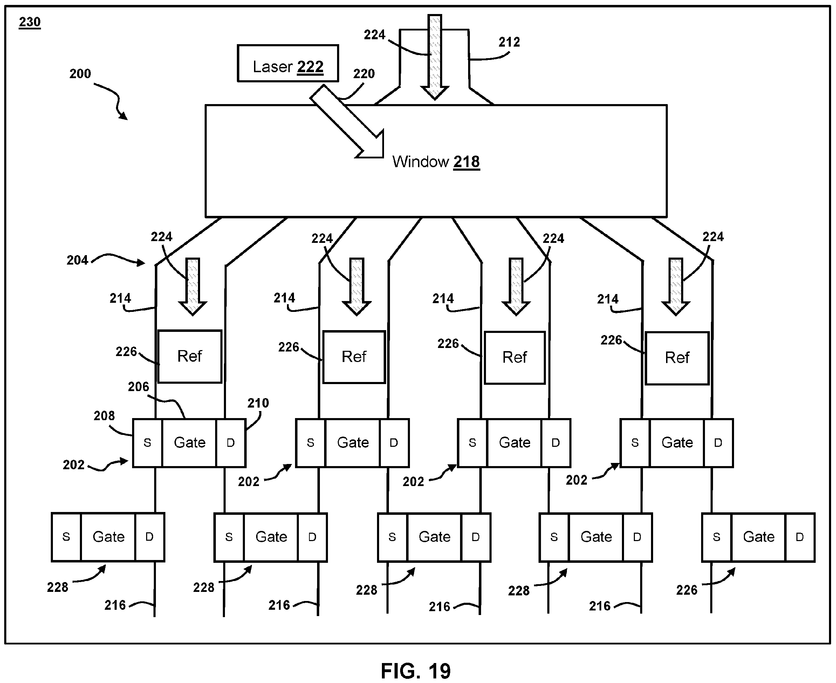

At process P2, the source to drain current I_{SD} of the ISFET 110 is measured. At process P3, the gate electrode 114 is pulse biased to attract additional ions 150 to the vicinity of the channel region 122 of the ISFET 110. At process P4, the source to drain current I_{SD} of the ISFET 110 is again measured. At process P5, the pulse biasing of the gate electrode 114 is terminated. At process P6, the source to drain current I_{SD} of the ISFET 110 is once again measured. At process P7, the frequency and excitation energy of the light 136 produced by the light source 138 may be adjusted (e.g., increased). Flow then passes back to process P1. Processes P1-P7 may then be repeated as many times as desired. In the case that the ISFET 110 does not include a planar gate 111, only the ions 150 in the microfluidic cavity 140 affect the source to drain current I_{SD} of the ISFET 110. To this extent, processes P3-P6 are not required. In some cases, a reference electrode (see discussion below with regard to FIGS. 19-20) may optionally be provided in the microfluidic cavity 140 to charge and excite ions 150 in the sample 142 flowing through the microfluidic cavity 140.

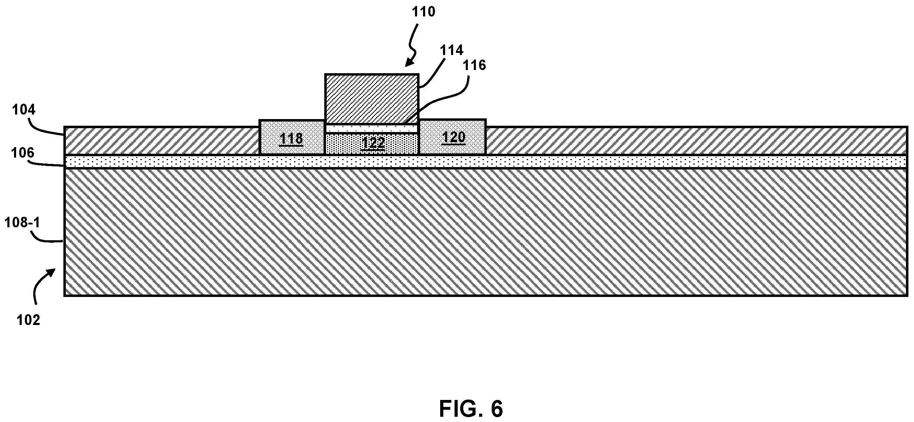

An illustrative process for forming the structure 100 of FIG. 1 according to embodiments of the disclosure is depicted in FIGS. 6-11.

In FIG. 6, an ISFET 110 is formed on a wafer 102 in a conventional manner (e.g., during FEOL processing). The ISFET 110 may include a source region 118, a drain region 120, and a channel region 122 positioned laterally between the source and drain regions 118, 120. The wafer 102 may be a silicon-on-insulator (SOI) wafer including a first substrate 108-1, an insulating layer 106, and a device layer 104.

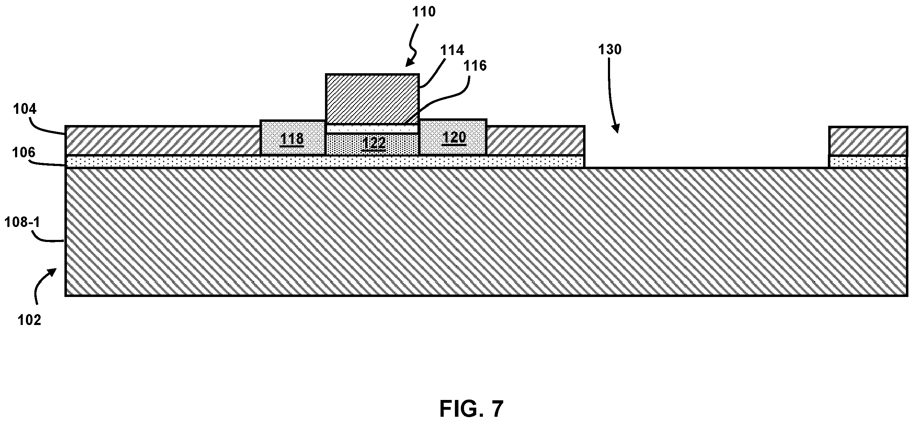

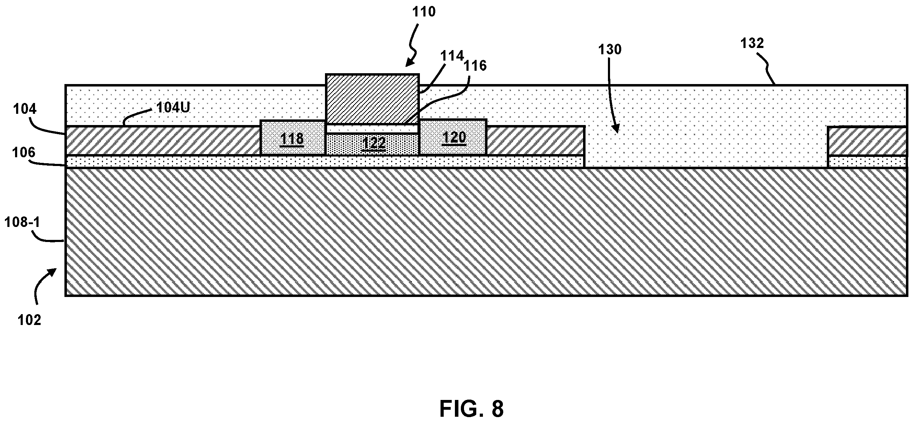

In FIG. 7, an opening 130 may be formed through a portion of the device layer 104 and the insulating layer 106 of the wafer 102 (e.g., during back-end-of-line (BEOL) processing) to form an opening 130. The opening 130 may extend to an upper surface of the first substrate 108-1. The opening 130 may be formed, for example, using known lithography and etching processes. In FIG. 8, after formation of the opening 130, a layer of insulating material 132 may be deposited over the ISFET 110, the upper surfaces 104U of the device layer 104, and the opening 130.

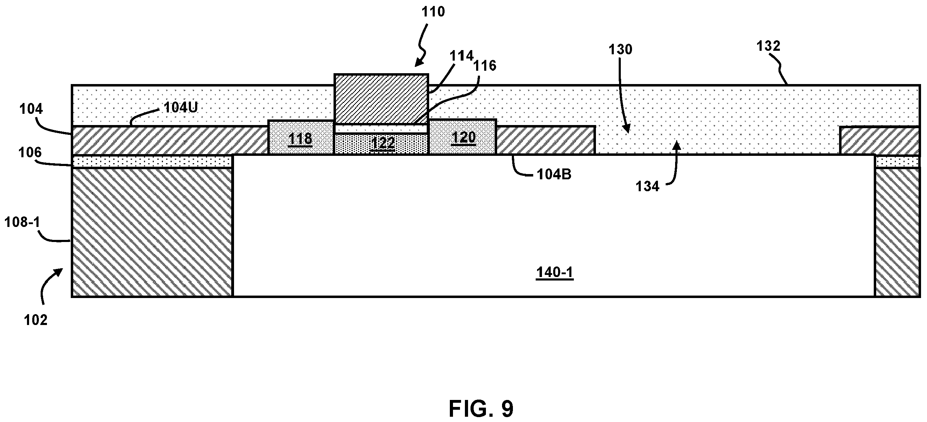

In FIG. 9, after deposition of the layer of insulating material 132, a first portion 140-1 of a microfluidic cavity 140 may be formed below the ISFET 110 and the opening 130 (e.g., via a back-side etch of the wafer 102) using known lithography and etching processes. As shown, the first portion 140-1 of the microfluidic cavity 140 extends from a bottom surface of the first substrate 108-1 to a bottom surface 104B of the device layer 104. As detailed above with regard to FIG. 1, the insulating material 132 within the opening 130 forms an insulating material window 134 that allows light 136 from a light source 138 to enter the microfluidic cavity 140 to allow excitation spectroscopy to be performed on ions in a sample 142 (e.g., fluid or gas) contained and/or flowing through the microfluidic cavity 140.

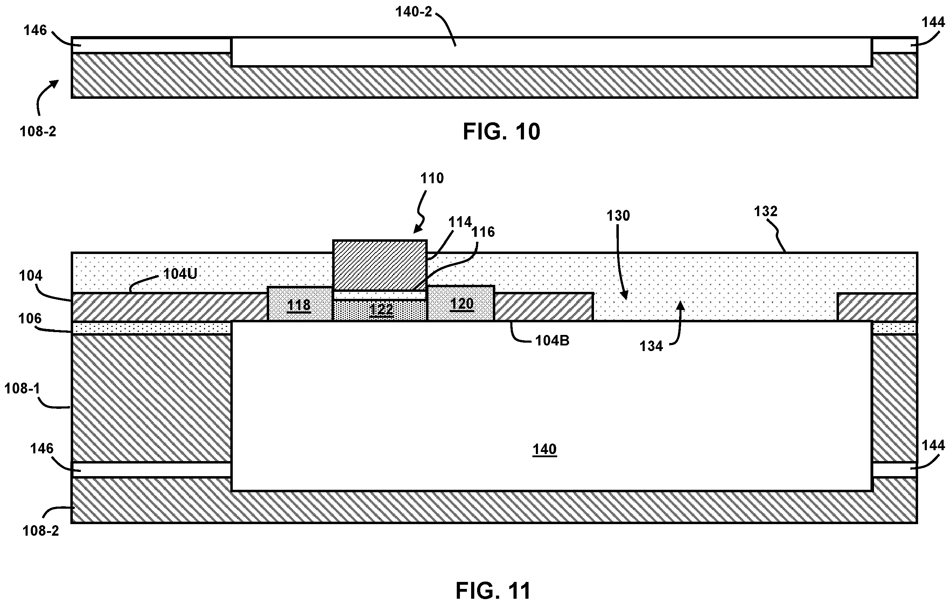

In FIG. 10, a second substrate 108-2 may be etched to form a second portion 140-2 of the microfluidic cavity 140. The second substrate 108-2 may also be etched to form one or more sample inlets 144, sample outlets 146, and/or other passages for passing a sample 142 into and out of the microfluidic cavity 140. Alternatively, or in addition, the first substrate 108-1 may be etched to form one or more sample inlets 144, sample outlets 146, and/or other passages for passing a sample 142 into and out of the microfluidic cavity 140. The number, size, shape, and placement of the sample inlets 144, sample outlets 146, and/or other passages should be sufficient to ensure that an adequate amount of the sample 142 can flow into and out of the cavity 140. Regardless, in FIG. 11, the second substrate 108-2 may be bonded in a known manner to the bottom of the first substrate 108-1 to form the semiconductor structure 100.

The structure 100 according to embodiments of the disclosure may also be formed using single wafer microfluidic processing. In this case, after forming the cavity 140 using a backside etch, the cavity 140 is filled with a sacrificial dielectric material and the exposed surface of the sacrificial dielectric material is planarized (e.g., using chemical mechanical planarization (CMP)). A cap, for example, of polycrystalline silicon, may then be formed over the sacrificial dielectric material. A wet etch may then be performed, via an inlet and outlet, to remove the sacrificial dielectric material from within the cavity 140.

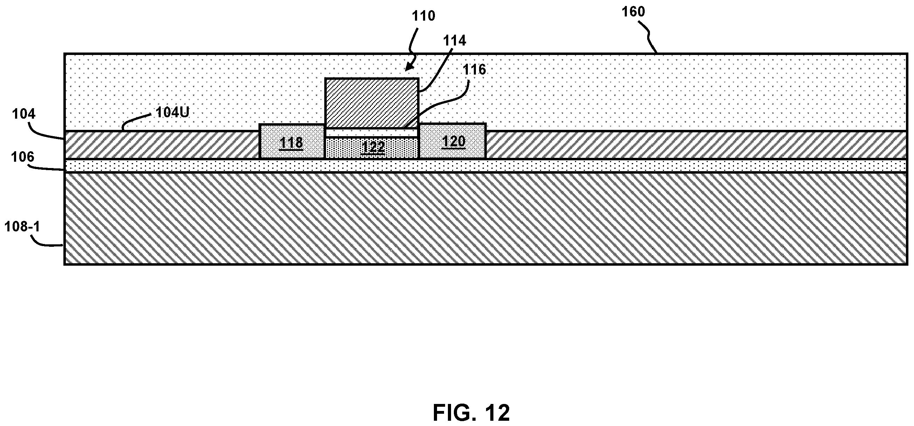

An illustrative process for forming the structure 100 of FIG. 3 according to embodiments of the disclosure is depicted in FIGS. 12-18.

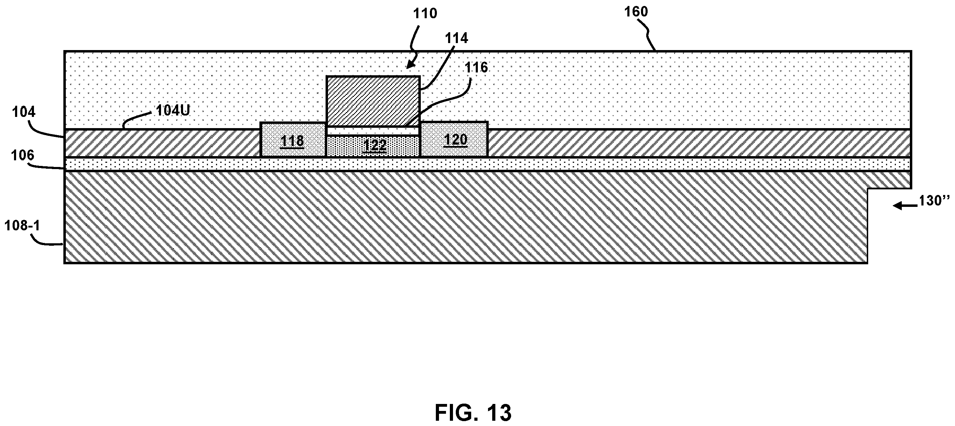

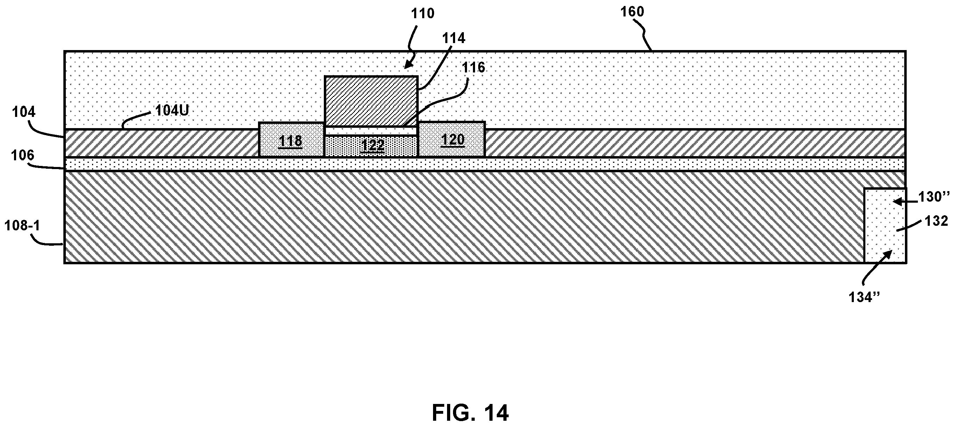

FIG. 12 depicts the structure 100 of FIG. 6 after a layer of an insulating material 160 has been formed over the ISFET 110 and the exposed upper surface 104U of the device layer 104. The insulating material 160 is formed to protect the ISFET 110 during subsequent processing. The insulating material 160 may be formed of silicon dioxide, silicon nitride, or other suitable insulating material, using a conformal deposition process or the like. An opening 130" may then be formed in a side of the first substrate 108-1 as depicted in FIG. 13 (e.g., during BEOL processing). The opening 130" may be formed, for example, using known lithography and etching processes. In FIG. 14, after formation of the opening 130", a layer of insulating material 132 may be deposited to fill the opening 130" to form an insulating material window 134". CMP may then be performed to planarize the exposed surfaces of the insulating material 132.

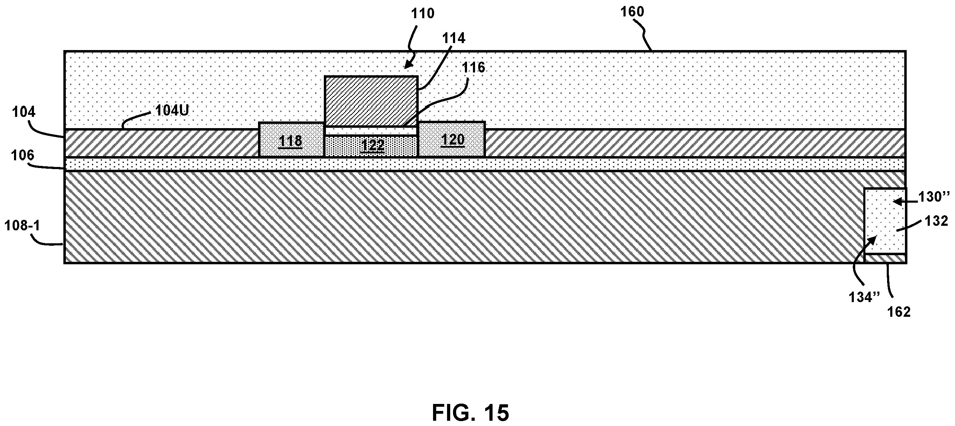

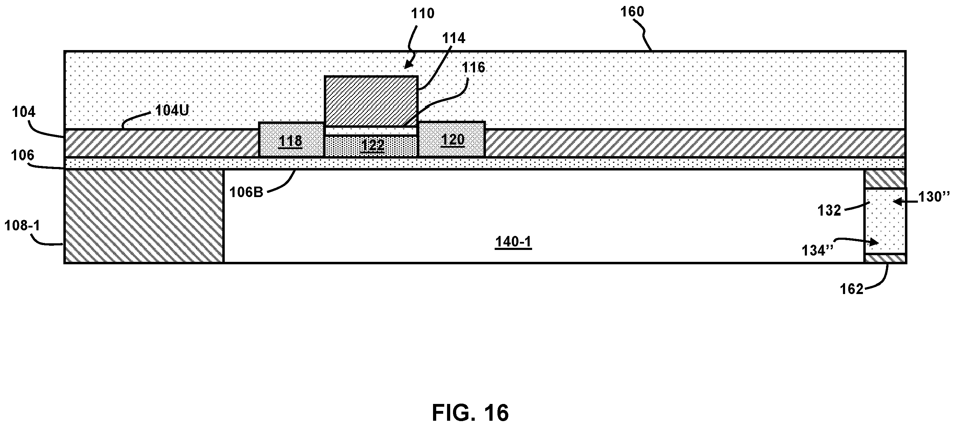

As depicted in FIG. 15, a cap 162, for example, of polycrystalline silicon, may then be formed over the bottom surface of the insulating material 132. Thereafter, as depicted in FIG. 16, a first portion of a microfluidic cavity 140-1 may be formed below the ISFET 110 (e.g., via a back-side etch of the substrate 108) using known lithography and etching processes. As shown, the first portion of the microfluidic cavity 140-1 extends from a bottom surface of the first substrate 108-1 to a bottom surface 106B of the insulating layer 106.

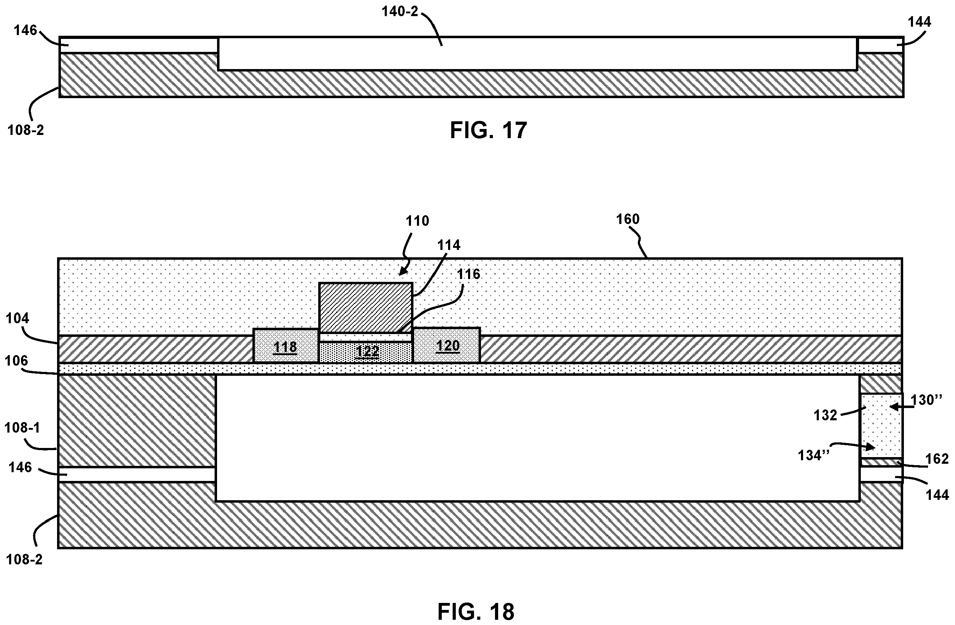

As depicted in FIG. 17, a second substrate 108-2 may be etched to form a second portion of the microfluidic cavity 140-2 and one or more sample inlets 144, sample outlets 146, and/or other passages for passing a sample 142 into and out of the microfluidic cavity 140. Thereafter, as shown in FIG. 18, the second substrate 108-2 may be bonded in a known manner to the bottom of the first substrate 108-1 to form the semiconductor structure 100.

FIG. 19 depicts a plan view of a semiconductor structure 200 including a plurality of ISFETS 202 and a microfluidic network 204 according to embodiments of the disclosure. Similar to the ISFET 110 depicted in FIG. 1, each ISFET 202 includes a planar gate 206 including a gate electrode and a gate dielectric (not shown), a source region 208, a drain region 210, and a channel region (not shown) positioned laterally between the source and drain regions 208, 210.

The microfluidic network 204 is formed in a semiconductor wafer 230 and includes a sample inlet 212 and a plurality of microfluidic cavities 214 branching from the sample inlet 212. An ISFET 202 is positioned over each microfluidic cavity 214. Each microfluidic cavity 214 further includes a sample outlet 216. A single insulating material window 218 extends over the plurality of microfluidic cavities 214 and is configured to pass light 220 from a single light source 222 (e.g., a tunable laser) into each of the microfluidic cavities 212. The light 220 entering each microfluidic cavity 214 excites ions in a sample 224 (e.g., fluid or gas) contained and/or flowing through the microfluidic cavity 214 (e.g., flowing from the sample inlet 212 to the sample outlet 216 through the microfluidic cavity 214). To this extent, ion detection and identification may be performed using the ISFETs 202, for example, in accordance with the process depicted in FIG. 5. According to embodiments of the disclosure, the plurality of microfluidic cavities 214 may have the same dimensions or may have different dimensions to provide filtering by size (e.g., filtering based on the size of molecules in the sample 224).

A conventional reference electrode 226 may be optionally be provided in each microfluidic cavity 214. The reference electrodes 226 are configured to charge and further excite ions in the sample 224 flowing through the microfluidic cavities 214.

The semiconductor structure 200 may further include a plurality of reference FETs 228 that are positioned between adjacent microfluidic cavities 212, with the gate of each reference FET 228 formed on silicon and the source and drain regions extending over adjacent microfluidic cavities 214. According to embodiments of the disclosure, the source to drain current I_{SD} of the reference FETs 228 may be measured before and after exciting ions in the sample 224 flowing through the microfluidic cavities 214. The difference between the source to drain current I_{SD} with no ions in the sample 224 and with ions in the sample 224 after excitation can be compared and used, for example, to improve the sensitivity of the semiconductor structure 200.

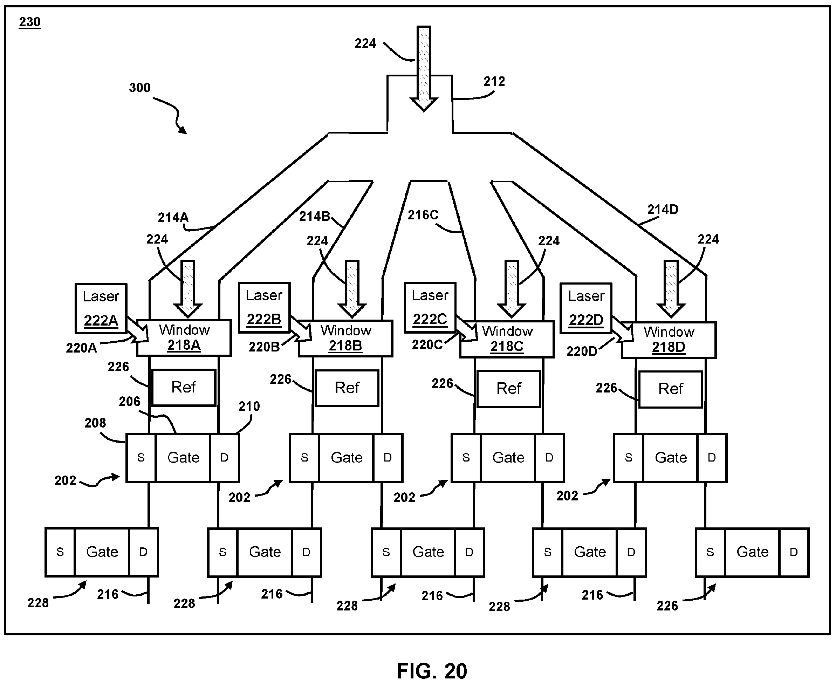

FIG. 20 depicts a plan view of a semiconductor structure 300 including a plurality of ISFETS 202 and a microfluidic network 204 according to embodiments of the disclosure. The semiconductor structure 300 is similar to the semiconductor structure 200 depicted in FIG. 19 and similar reference numbers are used to refer to similar elements. Unlike the semiconductor structure 200 however, which includes a single insulating material window 218 that extends over the plurality of microfluidic cavities 214 and which is configured to pass light 220 from a single light source 222 into each of the microfluidic cavities 212, the semiconductor structure 300 provides a separate insulating material window 218 (e.g., 218A-218D) over each microfluidic cavity 214 (e.g., 214A-214D). Further, separate light sources 222 (e.g., 222A-222D) may be provided to generate the light 220 (e.g., 220A-220D) that is passed though the insulating material windows 218 into the microfluidic cavities 214. In this way, light 220 of different frequencies may be used to excite samples 224 flowing through different microfluidic cavities 214. For example, the light source 222A may generate light 220A at a first frequency F₁, which is passed through the insulating material window 218A into microfluidic cavity 214A, the light source 222B may generate light 220B at a second, different frequency f₂, which is passed through the insulating material window 218B into microfluidic cavity 214B, and so one.

It should be understood that in the structures and methods described above, a semiconductor material refers to a material whose conducting properties can be altered by doping with an impurity. Exemplary semiconductor materials include, for example, silicon or germanium-based semiconductor materials (e.g., silicon, silicon germanium, silicon germanium carbide, silicon carbide, etc.) and III-V compound semiconductors (i.e., compounds obtained by combining group III elements, such as aluminum (Al), gallium (Ga), or indium (In), with group V elements, such as nitrogen (N), phosphorous (P), arsenic (As) or antimony (Sb)) (e.g., GaN, InP, GaAs, or GaP). A pure semiconductor material and, more particularly, a semiconductor material that is not doped with an impurity for the purposes of increasing conductivity (i.e., an undoped semiconductor material) is referred to in the art as an intrinsic semiconductor. A semiconductor material that is doped with an impurity for the purposes of increasing conductivity (i.e., a doped semiconductor material) is referred to in the art as an extrinsic semiconductor and will be more conductive than an intrinsic semiconductor made of the same base material. That is, extrinsic silicon will be more conductive than intrinsic silicon; extrinsic silicon germanium will be more conductive than intrinsic silicon germanium; and so on. Furthermore, it should be understood that different impurities (i.e., different dopants) can be used to achieve different conductivity types (e.g., P-type conductivity and N-type conductivity) and that the dopants may vary depending upon the different semiconductor materials used. For example, a silicon or germanium-based semiconductor material (e.g., silicon, silicon germanium, silicon germanium carbide, silicon carbide, etc.) is typically doped with a Group III dopant, such as boron (B) or indium (In), to achieve P-type conductivity, whereas such a semiconductor material is typically doped with a Group V dopant, such as arsenic (As), phosphorous (P) or antimony (Sb), to achieve N-type conductivity. A gallium nitride (GaN)-based semiconductor material is typically doped with magnesium (Mg) to achieve P-type conductivity and with silicon (Si) or oxygen to achieve N-type conductivity. Those skilled in the art will also recognize that different conductivity levels will depend upon the relative concentration levels of the dopant(s) in a given semiconductor region. Furthermore, when a semiconductor region or layer is described as being at a higher conductivity level than another semiconductor region or layer, it is more conductive (less resistive) than the other semiconductor region or layer; whereas, when a semiconductor region or layer is described as being at a lower conductivity level than another semiconductor region or layer, it is less conductive (more resistive) than that other semiconductor region or layer.

The method as described above is used in the fabrication of integrated circuit chips. The resulting integrated circuit chips can be distributed by the fabricator in raw wafer form (that is, as a single wafer that has multiple unpackaged chips), as a bare die, or in a packaged form. In the latter case the chip is mounted in a single chip package (such as a plastic carrier, with leads that are affixed to a motherboard or other higher level carrier) or in a multichip package (such as a ceramic carrier that has either or both surface interconnections or buried interconnections). In any case the chip is then integrated with other chips, discrete circuit elements, and/or other signal processing devices as part of either (a) an intermediate product, such as a motherboard, or (b) an end product. The end product can be any product that includes integrated circuit chips, ranging from toys and other low-end applications to advanced computer products having a display, a keyboard or other input device, and a central processor.

It should be understood that the terminology used herein is for the purpose of describing the disclosed structures and methods and is not intended to be limiting. For example, as used herein, the singular forms "a", "an" and "the" are intended to include the plural forms as well, unless the context clearly indicates otherwise. Additionally, as used herein, the terms "comprises" "comprising", "includes" and/or "including" specify the presence of stated features, integers, steps, operations, elements, and/or components, but do not preclude the presence or addition of one or more other features, integers, steps, operations, elements, components, and/or groups thereof. Furthermore, as used herein, terms such as "right", "left", "vertical", "horizontal", "top", "bottom", "upper", "lower", "under", "below", "underlying", "over", "overlying", "parallel", "perpendicular", etc., are intended to describe relative locations as they are oriented and illustrated in the drawings (unless otherwise indicated) and terms such as "touching", "in direct contact", "abutting", "directly adjacent to", "immediately adjacent to", etc., are intended to indicate that at least one element physically contacts another element (without other elements separating the described elements). The term "laterally" is used herein to describe the relative locations of elements and, more particularly, to indicate that an element is positioned to the side of another element as opposed to above or below the other element, as those elements are oriented and illustrated in the drawings. For example, an element that is positioned laterally adjacent to another element will be beside the other element, an element that is positioned laterally immediately adjacent to another element will be directly beside the other element, and an element that laterally surrounds another element will be adjacent to and border the outer sidewalls of the other element. The corresponding structures, materials, acts, and equivalents of all means or step plus function elements in the claims below are intended to include any structure, material, or act for performing the function in combination with other claimed elements as specifically claimed.

References herein to terms modified by language of approximation, such as "about", "approximately", and "substantially", are not to be limited to the precise value specified. The language of approximation may correspond to the precision of an instrument used to measure the value and, unless otherwise dependent on the precision of the instrument, may indicate +/-10% of the stated value(s).

References herein to terms such as "vertical", "horizontal", etc. are made by way of example, and not by way of limitation, to establish a frame of reference. The term "horizontal" as used herein is defined as a plane parallel to a conventional plane of a semiconductor substrate, regardless of its actual three-dimensional spatial orientation. The terms "vertical" and "normal" refer to a direction perpendicular to the horizontal, as just defined. The term "lateral" refers to a direction within the horizontal plane.

A feature "connected" or "coupled" to or with another feature may be directly connected or coupled to or with the other feature or, instead, one or more intervening features may be present. A feature may be "directly connected" or "directly coupled" to or with another feature if intervening features are absent. A feature may be "indirectly connected" or "indirectly coupled" to or with another feature if at least one intervening feature is present. A feature "on" or "contacting" another feature may be directly on or in direct contact with the other feature or, instead, one or more intervening features may be present. A feature may be "directly on" or "in direct contact with" another feature if intervening features are absent. A feature may be "indirectly on" or "in indirect contact with" another feature if at least one intervening feature is present.

The descriptions of the various embodiments of the present invention have been presented for purposes of illustration, but are not intended to be exhaustive or limited to the embodiments disclosed. Many modifications and variations will be apparent to those of ordinary skill in the art without departing from the scope and spirit of the described embodiments. The terminology used herein was chosen to best explain the principles of the embodiments, the practical application or technical improvement over technologies found in the marketplace, or to enable others of ordinary skill in the art to understand the embodiments disclosed herein.

In summary, the following embodiments are disclosed.
Embodiment 1:
   A structure, comprising: a cavity in a semiconductor substrate; an ion-sensitive field effect transistor (ISFET) positioned over the cavity; an opening in the semiconductor substrate extending to the cavity; and a layer of insulating material filling the opening and forming an insulating material window to the cavity.
Embodiment 2:
   The structure according to embodiment 1, wherein the opening is in any one of a top, bottom, and side of the semiconductor wafer.
Embodiment 3:
   The structure according to embodiment 1 or 2, wherein the insulating material window directs light from a light source into the cavity.
Embodiment 4:
   The structure according to embodiment 3, wherein the light source includes a tunable laser.
Embodiment 5:
   The structure according to one of embodiments 1 to 4, wherein the ISFET includes a drain region, a source region, and a channel region, and wherein the cavity extends to a bottom surface of the drain region, the source region, and the channel region of the ISFET.
Embodiment 6:
   The structure according to one of embodiments 1 to 5, wherein the cavity further includes at least one inlet and at least one outlet for passing a sample into and out of the cavity.
Embodiment 7:
   The structure according to one of embodiments 1 to 6, further comprising an insulating layer on the substrate, and a device layer on the insulating layer, wherein the cavity extends through the substrate and the insulating layer to a bottom surface of the device layer, and wherein the opening extends through the device layer to the cavity.
Embodiment 8:
   A method, comprising: directing light at a predetermined frequency through an insulating material window into a cavity in a semiconductor substrate, wherein the cavity contains a sample; and sensing a source to drain current of an ion-sensitive field effect transistor (ISFET) positioned over the cavity while directing the light at the predetermined frequency through the insulating material window into the cavity.
Embodiment 9:
   The method of embodiment 8, wherein the ISFET includes a planar gate, the sensing including: measuring the source to drain current of the ISFET; applying a pulse bias to a gate of the ISFET; measuring the source to drain current of the ISFET while applying the pulse bias to the gate of the ISFET; terminating the pulse bias to the gate of the ISFET; and measuring the source to drain current of the ISFET after terminating the pulse bias to the gate of the ISFET.
Embodiment 10:
   The method according to embodiment 8 or 9, further comprising: adjusting a frequency of the light; and measuring the source to drain current of the ISFET positioned over the cavity while directing the light at the adjusted frequency through the insulating material window into the cavity.
Embodiment 11:
   The method of one of embodiments 8 to 10, further comprising identifying ions in the sample based on the measured source to drain current of the ISFET.
Embodiment 12:
   The method of one of embodiments 8 to 11, further comprising correlating an excitation energy of the light to the measured source to drain current of the ISFET.
Embodiment 13:
   A structure comprising: a plurality of cavities in a semiconductor substrate; a plurality of ion-sensitive field effect transistors (ISFETs), wherein each ISFET of the plurality of ISFETS is positioned over a respective cavity of the plurality of cavities; and at least one insulating material window extending to the plurality of cavities, wherein the at least one insulating material window directs light from at least one light source into the plurality of cavities.
Embodiment 14:
   The structure according to embodiment 13, wherein the at least one insulating material window comprises a single insulating window extending to all of the plurality of cavities.
Embodiment 15:
   The structure according to embodiment 13 or 14, wherein the plurality of cavities have different dimensions.
Embodiment 16:
   The structure according to one of embodiments 13 to 15, wherein the at least one insulating material window comprises a plurality of insulating material windows, and wherein each insulating material window of the plurality of insulating material windows extends to a respective cavity of the plurality of cavities.
Embodiment 17:
   The structure according to embodiment 16, wherein the at least one light source comprises a plurality of light sources, and wherein each light source of the plurality of light sources directs light through a respective insulating material window of the plurality of insulating material windows into a respective cavity of the plurality of cavities.
Embodiment 18:
   The structure according to embodiment 17, wherein each light source of the plurality of light sources produces light at a different frequency.
Embodiment 19:
   The structure according to one of embodiments 13 to 18, wherein each cavity of the plurality of cavities further includes at least one inlet and at least one outlet for passing a sample into and out of the cavity.
Embodiment 20:
   The structure according to one of embodiments 13 to 19, wherein each ISFET of the plurality of ISFETs includes a drain region, a source region, and a channel region, wherein the cavity positioned under the ISFET extends to a bottom surface of the drain region, the source region, and the channel region of the ISFET. In other words, each ISFET of the plurality of ISFETs includes a drain region, a source region, and a channel region, and the cavity positioned under each ISFET extends to a bottom surface of the drain region, the source region, and the channel region of the respective ISFET.

## Claims

1. A structure, comprising:
at least one cavity in a semiconductor substrate;
at least one ion-sensitive field effect transistor (ISFET) positioned over the at least one cavity;
at least one opening in the semiconductor substrate extending to the at least one cavity;
and
a layer of insulating material filling the opening and forming at least one insulating material window to the at least one cavity.

2. The structure according to claim 1, wherein the at least one opening is in any one of a top, bottom, and side of the semiconductor wafer.

3. The structure according to claim 1 or 2, wherein the at least one insulating material window directs light from at least one light source into the at least one cavity, the at least one light source preferably including a tunable laser.

4. The structure according to one of claims 1 to 3, wherein each ISFET of the at least one ISFET includes a drain region, a source region, and a channel region, wherein the cavity positioned under each ISFET extends to a bottom surface of the drain region, the source region, and the channel region of the respective ISFET.

5. The structure according to one of claims 1 to 4, wherein the at least one cavity further includes at least one inlet and at least one outlet for passing a sample into and out of the cavity.

6. The structure according to one of claims 1 to 5, further comprising an insulating layer on the substrate, and a device layer on the insulating layer, wherein the at least one cavity extends through the substrate and the insulating layer to a bottom surface of the device layer, and wherein the at least one opening extends through the device layer to the at least one cavity.

7. The structure of one of claims 1 to 6, wherein the structure comprises:
a plurality of cavities in the semiconductor substrate; and
a plurality of ion-sensitive field effect transistors (ISFETs), wherein each ISFET of the plurality of ISFETS is positioned over a respective cavity of the plurality of cavities,
wherein the at least one insulating material window extends to the plurality of cavities, the at least one insulating material window directing light from at least one light source into the plurality of cavities.

8. The structure according to claim 7, wherein the at least one insulating material window comprises a single insulating window extending to all of the plurality of cavities, or wherein the at least one insulating material window comprises a plurality of insulating material windows, and wherein each insulating material window of the plurality of insulating material windows extends to a respective cavity of the plurality of cavities.

9. The structure according to claim 7, wherein the at least one insulating material window comprises a plurality of insulating material windows, and wherein each insulating material window of the plurality of insulating material windows extends to a respective cavity of the plurality of cavities, wherein the at least one light source comprises a plurality of light sources, and wherein each light source of the plurality of light sources directs light through a respective insulating material window of the plurality of insulating material windows into a respective cavity of the plurality of cavities, each light source of the plurality of light sources preferably producing light at a different frequency.

10. The structure according to one of claims 7 to 9, wherein the plurality of cavities have different dimensions, and/or wherein each cavity of the plurality of cavities further includes at least one inlet and at least one outlet for passing a sample into and out of the cavity.

11. A method, comprising:
directing light at a predetermined frequency through an insulating material window into a cavity in a semiconductor substrate, wherein the cavity contains a sample; and
sensing a source to drain current of an ion-sensitive field effect transistor (ISFET) positioned over the cavity while directing the light at the predetermined frequency through the insulating material window into the cavity.

12. The method of claim 11, wherein the ISFET includes a planar gate, the sensing including:
measuring the source to drain current of the ISFET;
applying a pulse bias to a gate of the ISFET;
measuring the source to drain current of the ISFET while applying the pulse bias to the gate of the ISFET;
terminating the pulse bias to the gate of the ISFET; and
measuring the source to drain current of the ISFET after terminating the pulse bias to the gate of the ISFET.

13. The method according to claim 11 or 12, further comprising:
adjusting a frequency of the light; and
measuring the source to drain current of the ISFET positioned over the cavity while directing the light at the adjusted frequency through the insulating material window into the cavity.

14. The method of one of claims 11 to 13, further comprising identifying ions in the sample based on the measured source to drain current of the ISFET.

15. The method of one of claims 11 to 14, further comprising correlating an excitation energy of the light to the measured source to drain current of the ISFET.
